# EUROPEAN PATENT SPECIFICATION

(11) **EP 1 335 699 B1**
(45) Date of publication and mention of the grant of the patent: **22.03.2006**
(21) Application number: 01979475.9
(22) Date of filing: 05.10.2001
(51) Int. Cl.: A61K 8/04, A61K 8/29, A61K 8/35, A61K 8/37, A61K 8/40, A61K 8/91, A61Q 17/04, A61Q 19/04

(54) **CONCORDANTLY UV-PHOTOPROTECTING AND ARTIFICIAL TANNING COMPOSITIONS**
ZUSAMMENSETZUNGEN ZUM GLEICHZEITIGEN UV-SCHUTZ UND KÜNSTLICHER BRÄUNUNG
COMPOSITIONS ASSURANT SIMULTANEMENT UNE PHOTOPROTECTION CONTRE LES UV ET UN BRONZAGE ARTIFICIEL

(30) Priority: 05.10.2000 US 237703 P
(43) Date of publication of application: 20.08.2003
(73) Proprietor: Societe L'Oreal S.A., 75008 Paris (FR)
(72) Inventor: HANSENNE, Isabelle, Westfield, NJ 07090 (US); FEENEY, Brendan, Brick, NJ 08732 (US); GALDI, Angelike, Westfield, NJ 07090 (US)
(74) Representative: Miszputen, Laurent
(86) International application number: PCT/US2001/031146
(87) International publication number: WO 2002/028359

(56) References cited:
- WO-A-95/06079
- US-A- 5 567 428
- US-A- 5 951 967

## Description

### BACKGROUND OF THE INVENTION

### Technical Field of the Invention:

The present invention relates to novel dermatological compositions for topical application, for the ultraviolet (UV)-photoprotection (typically providing enhanced sun protection factors (SPF)) and artificial tanning and/or darkening of mammalian skin and/or scalp.

The present invention more especially relates to the aforesaid concordantly UV-photoprotecting/artificial tanning compositions comprising stable intimate immixture of a sunscreen, a self-tanning agent, and a judiciously selected silicone/acrylate graft copolymer.

### Description of the Prior Art:

It is known to this art that light radiation of wavelengths of from 280 nanometers ("nm") to 400 nm permits tanning of the human epidermis and that light radiation of wavelengths of from 280 to 320 nm, i.e., UV-B irradiation, causes skin burns and erythema which may be harmful to the development of a natural tan; this UV-B radiation should therefore be screened from the skin.

It is also known to this art that UV-A radiation, of wavelengths of from 320 to 400 nm, promotes tanning of the skin, but is also liable to induce an adverse change therein, especially in the case of sensitive skin or skin which is continually exposed to solar radiation. UV-A rays, in particular, cause a loss of elasticity of the skin and the appearance of wrinkles which promotes a premature aging of the skin. Such irradiation further promotes triggering of the erythemal reaction or amplifies this reaction in certain individuals and may even be the cause of phototoxic or photoallergic reactions. It is thus desirable to also screen out UV-A radiation.

It is also known to this art that allose, alpha hydroxy substituted ketones such as dihydroxyacetone, altrose, arabinose, erythrose, fructose, galactose, glucose, glyceraldehyde, indoles, lactose, mannose, reose, ribose, pentose, sucrose, tallose, xylose, and derivatives and combinations thereof are particularly advantageous compounds which may be employed in cosmetics as an agent for the artificial tanning of mammalian skin. When applied to mammalian skin, these agents elicit a tanning or darkening effect whose appearance is similar to that which may result from prolonged exposure to the sun or a UV lamp. When used as such, it presents the advantage of eliminating the need to encounter the skin or cutaneous reactions which are generally associated with the aforesaid prolonged exposures.

It is known in this art that many persons who employ the benefits of artificial tanning compositions do so solely for aesthetic reasons. It is also known in this art that many persons who employ the benefits of artificial tanning compositions do so in an effort to achieve a tanned appearance without having encountered the harmful exposure to the sun. In both cases, it is most likely that these persons will inevitably encounter harmful exposure to the sun. Therefore, the need exists for cosmetic/dermatological compositions which provide an effective artificial tan as well as effective UV-photoprotection.

One of the most desirable and effective artificial tanning agents is the alpha hydroxy substituted ketone, dihydroxyacetone, or "DHA". Unfortunately, DHA has many inherent disadvantages. DHA is known to produce a somewhat unnatural orange color on the skin. DHA is highly reactive with other ingredients of a mixture, decomposes rapidly, and has an odor which is not acceptable for cosmetics. Moreover, DHA takes an unacceptably long time to color the skin which then fades in too short a time.

To this end, compositions have been described in the past which serve as both artificial tanners and UV-photoprotecting agents and seek to overcome the disadvantages of DHA. U.S. Patent No. 6,007,796, issued to Menzel et al. on December 28, 1999, describes a cosmetic self-tanning agent based on DHA which also has a sunscreen effect. The object of the patent is to provide a combination of sunscreen and an agent in which the self-tanning effect occurs shortly after application and over a long period of time, and in which a plurality of color shades may be obtained without additional color components. However, this cosmetic achieves only an SPF of six to ten.

International Patent Publication No. WO 94/23693 describes a composition which is a combination of tanning agent based on DHA and sunscreen. The composition contains 0.1 % to 20% DHA, 0.1 % to 30% of a sunscreen, 0.1 % to 10% of a cross-linked cationic polymer, and 0.1% to 10% of a cationic emulsifier.

International Patent Publication No. WO 95/06079 generally describes various compositions, including a possible artificial tanning and UV-photoprotecting composition, but containing a characteristic polymer which is water soluble and is of a distinctly different chemical structure than the present invention.

To date, no combination UV-photoprotecting/artificial tanning compositions have achieved acceptable SPF while maintaining stability.

It is an object of the present invention to provide novel cosmetic/dermatological compositions.

It is an object of the present invention to provide novel topically applicable cosmetic/dermatological compositions.

It is another object of the present invention to provide novel cosmetic/dermatological stable compositions having enhanced SPFs and maintained artificial tanning properties.

It is yet another object of this invention to provide novel cosmetic/dermatological stable compositions having acceptable and typically enhanced SPFs and maintained artificial tanning properties which include DHA.

It is still another object of this invention to provide novel cosmetic/dermatological compositions having acceptable and typically enhanced SPFs and maintained artificial tanning properties which contain DHA, which are stable, and which may be topically applied onto the skin via spray delivery.

It is yet another object of the present invention to provide a novel technique or regime/regimen for protecting mammalian skin and/or scalp against the deleterious effects of UV-radiation.

It too is an object of this invention to provide novel methodology for enhancing the stability and SPF value of a cosmetic/dermatological composition comprising an artificial tanning agent, especially DHA.

These and other objects will become readily apparent from the detailed description which follows.

### SUMMARY OF THE INVENTION

It has now unexpectedly and surprisingly been determined that concordantly UV-photoprotecting/artificial tanning compositions comprising poly(isobutyl methacrylate-co-methyl FOSEA)-g-poly(dimethylsiloxane), also known as "SA 70-5", provide an SPF that is acceptable and typically enhanced, but which are nevertheless easily formulated and quite stable over time.

It too has been determined that other silicone/acrylate graft copolymers, notably SA 70 "plus," a terpolymer of isobutyl methacrylate, acrylic acid and a methacryloxypropyl/butyldimethyl silyl macromer, afford essentially comparable results.

Thus, the present invention features novel dermatological compositions which comprise: (i) an effective UV-photoprotecting amount of at least one UV-photoprotecting agent, (ii) an effective artificial tanning amount of at least one artificial tanning agent, and (iii) a compatibilizing amount of a silicone/acrylate graft copolymer which comprises an acrylic backbone having pendant organosiloxane branches, particularly polysiloxane branches, depending therefrom in the form of a compatibilizing amount of poly(isobutyl methacrylate-co-methyl FOSEA)-g-poly(dimethylsiloxane) or of a terpolymer of isobutyl methacrylate, acrylic acid and a methacryloxypropyl/butyldimethyl silyl macromer.

The present invention also features a method for the preparation of a composition for artificially tanning and protecting mammalian skin and/or scalp against the deleterious effects of ultraviolet irradiation, comprising topically applying onto the skin and/or scalp a dermatological composition comprising: (i) an effective UV-photoprotecting amount of at least one UV-photoprotecting agent, (ii) an effective artificial tanning amount of at least one artificial tanning agent, and (iii) a compatibilizing amount of a silicone/acrylate graft copolymer which comprises an acrylic backbone having pendant organosiloxane branches, particularly polysiloxane branches, depending therefrom, in the form of a compatibilizing amount of poly(isobutyl methacrylate-co-methyl FOSEA)-g-poly(dimethylsiloxane) or of a terpolymer of isobutyl methacrylate, acrylic acid and a methacryloxypropyl/butyldimethyl silyl macromer.

Too, this invention also features methodology for stabilizing and typically SPF-enhancing a dermatological composition comprising at least one UV-photoprotecting agent and at least one artificial tanning agent, comprising formulating and admixing into said composition a compatibilizing amount of a silicone/acrylate graft copolymer which comprises an acrylic backbone having pendant organosiloxane branches, particularly polysiloxane branches, depending therefrom, and notably a compatibilizing/stabilizing amount of poly(isobutyl methacrylate-co-methyl FOSEA)-g-poly(dimethylsiloxane) or of a terpolymer of isobutyl methacrylate, acrylic acid and a methacryloxypropyl/butyldimethyl silyl macromer.

### DETAILED DESCRIPTION OF BEST MODE AND SPECIFIC/PREFERRED EMBODIMENTS OF THE INVENTION

The terms "artificial tanner" and "artificial tanning agent" as used herein may be used interchangeably, and refer to one or more active ingredients which, when applied to mammalian skin, elicit a tanning or darkening effect, the appearance of which is similar to that which may result from prolonged exposure to the sun or under a UV lamp.

The term "DHA" as used herein refers to the alpha hydroxy substituted ketone, dihydroxyacetone.

The term "graft copolymer" is a term familiar to those of ordinary skill in polymer sciences which describes copolymers onto the backbone of which another chemical moiety has been added, or "grafted."

The term "sun protection factor" is abbreviated by, and may be used interchangeably with, the term "SPF" and is the ratio of time of ultraviolet radiation (both UVA and UVB) exposure necessary to produce minimally detectable erythema in sunscreen-protected skin to that time for unprotected skin.

The terms "sunscreen" and "UV-photoprotecting agent" refer to one or more active ingredients which effectively either physically block or chemically absorb ultraviolet radiation in the region of the electromagnetic spectrum including wavelengths from 280 to 400 nm.

The terms "ultraviolet radiation" and "UV-radiation," which may be used interchangeably, as used herein refer to radiation in the region of the electromagnetic spectrum including wavelengths from 280 to 400 nm.

The term "UVA-radiation" as used herein refers to radiation in the region of the electromagnetic spectrum including wavelengths from 320 to 400 nm.

The term "UVB-radiation" as used herein refers to radiation in the region of the electromagnetic spectrum including wavelengths from 280 to 320 nm.

Sunscreens may be classified based upon the type of protection they provide. There are two major sunscreen classifications: physical blockers and chemical absorbers.

Sunscreens which contain physical blockers reflect or scatter ultraviolet radiation. Typical examples of physical blockers include red petrolatum, titanium dioxide, and zinc oxide. These physical blockers have been used in a variety of suspensions and particle sizes and are used frequently in cosmetic formulations. A review of physical blockers may be found at "Sun Protection Effect of Nonorganic Materials," by S. Nakada & H. Konishi, Fragrance Journal, Volume 15, pages 64-70 (1987).

Sunscreens which contain chemical absorbers actually absorb harmful ultraviolet radiation. Chemical absorbers are classified, depending on the type of radiation they protect against, as either UVA or UVB absorbers. UVA absorbers generally absorb radiation in the 320 to 400 nm region of the ultraviolet spectrum. UVA absorbers include anthranilates, benzophenones, and dibenzoyl methanes. UVB absorbers generally absorb radiation in the 280 to 320 nm region of the ultraviolet spectrum. UVB absorbers include *p*-aminobenzoic acid derivatives, camphor derivatives, cinnamates, and salicylates.

Classifying the chemical absorbers generally as UVA or UVB absorbers is accepted within the industry. However, a more precise classification is one based upon the chemical properties of the sunscreens. There are eight major classifications of sunscreen chemical properties which are discussed at length in "Sunscreens - Development, Evaluation and Regulatory Aspects," by N. Shaath et al., 2nd. Edition, pages 269-273, Marcel Dekker, Inc. (1997).

The sunscreens of the present invention typically comprise chemical absorbers, but may also comprise physical blockers. Among the sunscreens which are useful in the compositions of the present invention are chemical absorbers such as *p*-aminobenzoic acid derivatives, anthranilates, benzophenones, camphor derivatives, cinnamic derivatives, dibenzoyl methanes, β,β-diphenylacrylate derivatives, salicylic derivatives, triazine derivatives, benzimidazole compounds, bis-benzoazolyl derivatives, methylene bis-(hydroxyphenylbenzotriazole) compounds, the sunscreen polymers and silicones, or mixtures thereof. These are variously described in U.S. Patents Nos. 2,463,264, 4,367,390, 5,166,355 and 5,237,071 and in EP-0,863,145, EP-0,517,104, EP-0,570,838, EP-0,796,851, EP-0,775,698, EP-0,878,469, EP-0,933,376, EP-0,893,119, EP-0,669,323, GB-2,303,549, DE-1,972,184 and WO-93/04665. Among the sunscreens which are useful in the compositions of the present invention are physical blockers such as cerium oxides, chromium oxides, cobalt oxides, iron oxides, red petrolatum, silicone-treated titanium dioxide, titanium dioxide, zinc oxide, and/or zirconium oxide, or mixtures thereof.

A wide variety of sunscreens is described in U.S. Patent No. 5,087,445, issued to Haffey et al. on February 11, 1992; U.S. Patent No. 5,073,372, issued to Turner et al. on December 17, 1991; and Chapter VIII of Cosmetics and Science and Technology by Segarin et al. , pages 189 et seq. (1957).

Preferred among those sunscreens which are useful in the compositions of the instant invention are those selected from the group comprising: aminobenzoic acid, amyldimethyl PABA, cinoxate, diethanolamine *p-*methoxycinnamate, digalloyl trioleate, dioxybenzone, 2-ethoxyethyl *p-*methoxycinnamate, ethyl 4-bis(hydroxypropyl)aminobenzoate, 2-ethylhexyl-2-cyano-3,3-diphenylacrylate, ethylhexyl *p*-methoxycinnamate, 2-ethylhexyl salicylate, glyceryl aminobenzoate, homomenthyl salicylate, homosalate, 3-imidazol-4-ylacrylic acid and ethyl ester, methyl anthranilate, octyldimethyl PABA, 2-phenylbenzimidazole-5-sulfonic acid and salts, red petrolatum, sulisobenzone, titanium dioxide, triethanolamine salicylate, *N*, *N*, *N*-trimethyl-4-(2-oxoborn-3-ylidene methyl)anillinium methyl sulfate, and mixtures thereof.

Similarly preferred sunscreens active in the UV-A and/or UV-B range include:
p-aminobenzoic acid,
oxyethylene (25 mol) p-aminobenzoate,
2-ethylhexyl p-dimethylaminobenzoate,
ethyl N-oxypropylene p-aminobenzoate,
glycerol p-aminobenzoate,
4-isopropylbenzyl salicylate,
2-ethylhexyl 4-methoxycinnamate,
methyl diisopropylcinnamate,
isoamyl 4-methoxycinnamate,
diethanolamine 4-methoxycinnamate,
3-(4'-trimethylammunium)-benzyliden-bornan-2-one methylsulfate,
2-hydroxy-4-methoxybenzophenone,
2-hydroxy-4-methoxybenzophenone-5-sulfonate,
2,4-dihydroxybenzophenone,
2,2',4,4' -tetrahydroxybenzophenone,
2,2'-dihydroxy-4,4'dimethoxybenzophenone,
2-hydroxy-4-n-octoxybenzophenone,
2-hydroxy-4-methoxy-4'-methoxybenzophenone,
α-(2-oxoborn-3-ylidene)-tolyl-4-sulfonic acid and soluble salts thereof,
3-(4'-sulfo)benzyliden-bornan-2-one and soluble salts thereof,
3-(4'methylbenzylidene)-d,l-camphor,
3-benzylidene-d,l-camphor,
benzene 1,4-di(3-methylidene-10-camphosulfonic) acid and salts thereof (the product Mexoryl SX described in U.S. Patent No. 4,585,597 issued to Lange et al. on April 29, 1986),
urocanic acid,
2,4,6-tris[p-(2'-ethylhexyl-1'-oxycarbonyl)-anilino]-1,3,5-triazine,
2-[(p-(tertiobutylamido)anilino]-4,6-bis-[(p-(2'-ethylhexyl-1'-oxycarbonyl)anilino]-1,3,5-triazine,
2,4-bis{[4-(2-ethyl-hexyloxy)]-2-hydroxy]-phenyl}-6-(4-methoxy-phenyl)-1,3,5-triazine ("TINOSORB S" marketed by Ciba),
the polymer of N-(2 et 4)-[(2-oxoborn-3-yliden)methyl]benzyl]-acrylamide,
1,4-bisbenzimidazolyl-phenylen-3,3',5,5'-tetrasulfonic acid and salts thereof,
the benzalmalonate-substituted polyorganosiloxanes,
the benzotriazole-substituted polyorganosiloxanes (Drometrizole Trisiloxane),
solubilized 2,2'-methylene-bis-[6-(2H-benzotriazol-2-yl)-4-(1,1,3,3-tetramethylbutyl)phenol] such as that marketed under the trademark MIXXIM BB/100 by Fairmount Chemical, or micronized in soluble form thereof such as that marketed under the trademark TINOSORB M by Ciba-Geigy, and
solubilized 2,2'-methylene-bis-[6-(2H-benzotriazol-2-yl)-4-(methyl)phenol] such as that marketed under the trademark MIXXIM BB/200 by Fairmount Chemical.

Typically preferred among these sunscreens which are useful in the compositions of the present inventions are one or more of the following: avobenzone, octyl salicylate, octocrylene, and oxybenzone. Combinations of one of more of these sunscreens is similarly preferred.

The dibenzoyl methane derivatives are also particularly preferred sunscreens according to the present invention. These are described, for example, in FR-2,326,405, FR-2,440,933 and EP-0,114,607.

More particularly preferred dibenzoyl methane sunscreens include (whether singly or in any combination):
2-methyldibenzoylmethane
4-methyldibenzoylmethane
4-isopropyldibenzoylmethane
4-tert.-butyldibenzoylmethane
2,4-dimethyldibenzoylmethane
2,5-dimethyldibenzoylmethane
4,4'-diisopropyldibenzoylmethane
4,4'-dimethoxydibenzoylmethane
4-tert. -butyl-4' -m ethoxydibenzoylmethane
2-methyl-5-isopropyl-4'-methoxydibenzoylmethane
2-methyl-5-tert.-butyl-4'-methoxydibenzoylmethane
2,4-dimethyl-4'-methoxydibenzoylmethane
2,6-dimethyl-4-tert.-butyl-4'-methoxydibenzoylmethane

An especially preferred dibenzoyl methane sunscreen is 4-(tert-butyl),4'methoxydibenzoyl methane, particularly that marketed under the trademark "PARSOL 1789" by Givaudan, having the structural formula:

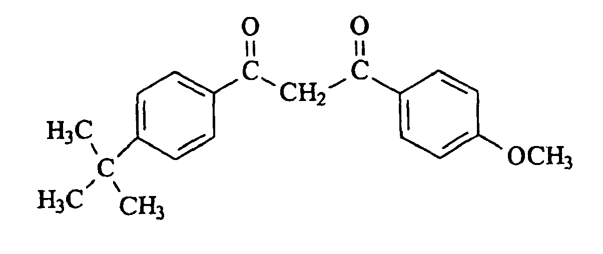

Another especially preferred dibenzoyl methane sunscreen is 4-isopropyldibenzoyl methane, particularly that marketed by Merck under the trademark "EUSOLEX 8020", having the structural formula:

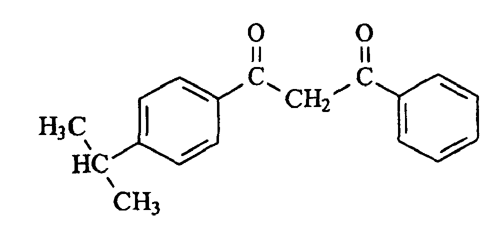

The dibenzoyl methane sunscreens are advantageously formulated into the compositions of the invention in amounts ranging from about 0.01 % to about 10%, and preferably from about 0.1 % to about 6%, by weight thereof. Of course, depending upon the nature of the particular formulation, higher or lower amounts may be suitable.

To date, a wide variety of artificial tanning agents has been developed. Artificial tanners provide the highly sought-after tanning or darkening response once only available through harmful exposure to ultraviolet radiation. DHA, in particular, has been widely utilized in cosmetics to accomplish artificial tanning of the skin. Proteins of the epidermis have a very high concentration of the arginine, lysine, and histidine and the reaction of skin with DHA to produce an artificial tan takes advantage of this fact. The tanning reaction proceeds through combination with free amino groups in skin proteins, and particularly by combination of DHA with the free guanido group in arginine.

Preferred among those artificial tanners which are useful in the compositions of the instant invention are those selected from the group comprising: allose, alpha hydroxy substituted ketones such as dihydroxyacetone, altrose, arabinose, erythrose, fructose, galactose, glucose, glyceraldehyde, indoles, lactose, mannose, reose, ribose, pentose, sucrose, tallose, xylose, and mixtures thereof.

Typically preferred among these artificial tanners which are useful in the compositions of the present inventions is dihydroxyacetone. In this respect, it should be appreciated that DHA is not at all easy to formulate, is particularly sensitive and compositions comprised thereof tend to be quite unstable over time (as DHA tolerates but few raw materials, e.g., carbomers). Thus, the stable formulations according to the invention, especially those suited for spray delivery, are all the more unexpected and surprising.

SA 70-5 is silicone acrylate polymer. Silicone acrylate polymers are graft copolymers with silicone branches attached to acrylic backbones. These graft copolymers show the properties of both chain components independently, as opposed to random copolymers, which show an average property. Silicone acrylate polymers provide silicone-like features such as water repellency, lubricity, flexibility, gloss, low softening temperature, chemical inertness and solubility or compatibility with organics. Silicone acrylate polymers also provide acrylic-like features such as durability, strong adhesion to substrates, variable brittleness, and the ability to combine into water and alcohol based formulations.

SA 70-5 has a combination of an acrylic monomer and a fluoromonomer in the backbone which provides a substantivity to substrates and water repellency. Once SA 70-5 is applied to a substrate, the solvent begins evaporating. The acrylic chains align themselves and silicones phase separate during this process. The acrylic chains bond to the polar substrate and the silicones migrate to the surface, which gives the strength of an acrylic adhesion to the substrate and the surface texture of a silicone. In a word, the silicone/acrylate graft copolymers of this invention present the silicone phase at the surface with the acrylate phase anchored to the skin.

SA 70-5 is a polymer having moieties of the general formula: 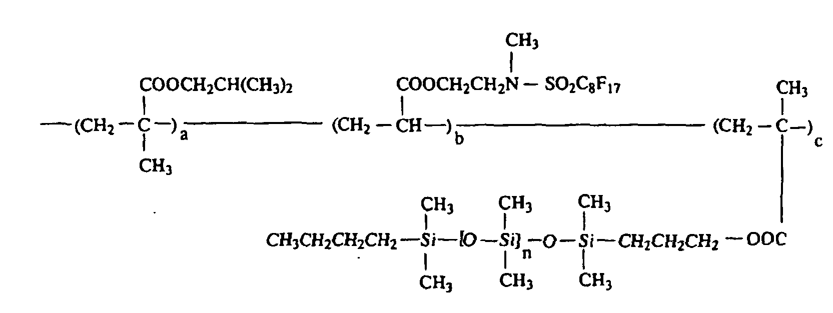 wherein a, b, and c are 1-100,000, and the terminal groups can be C₁₋₂₀ straight or branched chain alkyl, aryl, alkoxy, and the like. This polymer may be purchased from Minnesota Mining and Manufacturing Company. United States patents which disclose this polymer include U.S. Patent No. 4,972,037, issued to Garbe et al. on November 20, 1990; U.S. Patent No. 5,061,481, issued to Suzuki et al. on October 29, 1991; U.S. Patent No. 5,209,924, issued to Garbe et al. on May 11, 1993; and U.S. Patent No. 5,849,275, issued to Calello et al. on December 15, 1998. International Patent Publications which disclose this polymer include WO 93/23446 and WO 95/06078.

The CAS Registry Number of SA 70-5 is 146632-08-8; characteristically it has a molecular weight of 71,000 (polymer Mₙ polystyrene standard, THF, RI detector), a polydispersity of 3.4 and an inherent viscosity of 0.32 dl/g (polymer in ethyl acetate @ 25°C).

SA 70 "plus" is a silicone/acrylate graft terpolymer comprising recurring structural units having the formula: 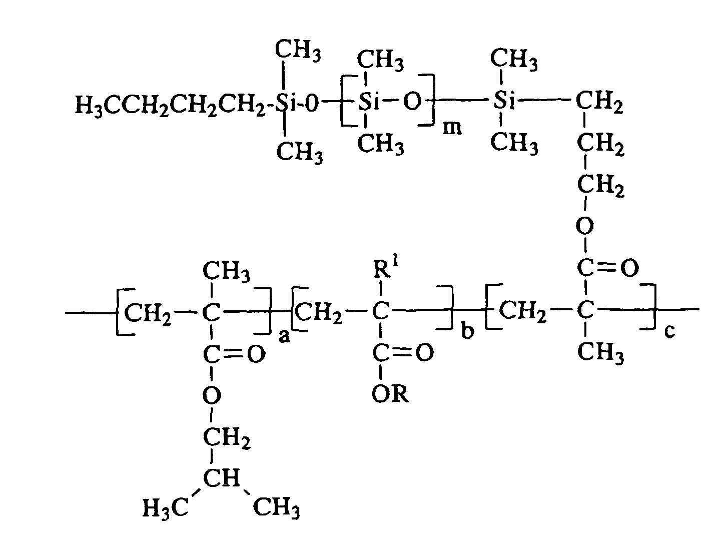 wherein the weight ratios of a, b and c are about 69.9, 0.1 and 30, respectively. R and R¹ are each hydrogen (but otherwise could be lower alkyl) and m is about 130 to provide a macromer molecular weight on the order of 10,000. These copolymers comprising silicone macromers are described in WO 01/32727 A1.

The copolymers of the present invention are present in the instant compositions in a "compatibilizing amount", i.e., in an amount sufficient to formulate same while maintaining an acceptable SPF and/or to stabilize the final composition. Typically, the copolymer comprises from about 0.5% to about 10% by weight, preferably from about 1 % to about 5 % by weight, and most preferably about 1 % by weight of the composition, although higher or lower amounts may be used depending on the particular formulation.

The SA 70-5 or polysilicone-7 polymer is available in solution (cyclomethicone). SA 70-5 is also available in Finsolv™ TNO (C12-15 alkyl benzoate), and in solid form. Each of these forms may be used in the compositions of the present invention.

The composition of the present invention can be formulated into a wide variety of product types, including creams, dispersions, emulsions (oil-in-water, water-in-oil, water-in-oil-in-water, and oil-in-water-in-silicone), gels, lotions, milks, mousses, sprays, tonics, and the like.

In an especially preferred embodiment of the present invention, the subject cosmetic/dermatological compositions are provided as spray delivery systems. This because, heretofore, it was particularly difficult to attain high SPFs with a spray. Also, because of its ease of use, the consumer particularly appreciates a spray delivery system.

The topical cosmetic compositions of the present invention typically comprise a carrier (vehicle or diluent) or mixture of carriers. The carrier should be cosmetically and/or pharmaceutically acceptable, which means that the carrier is suitable for topical application to the skin, has good aesthetic properties, is compatible with the copolymer of the present invention, and any other components, and will not cause any untoward safety or toxicity concerns. The carriers and additional components used to formulate such products vary with the product type and may be routinely chosen by one skilled in the art. The following is a description of some of these carriers and additional components.

The compositions of the present invention can comprise a carrier, or a mixture of carriers, which are suitable for application to mammalian skin. The carriers are present from about 0.5% to about 99.5% by weight, preferably from about 5.0% to about 99.5% by weight, more preferably from about 10.0% to about 98.0% by weight, of the composition. As used herein, the phrase "suitable for application to mammalian skin" means that the carrier does not damage or negatively affect the aesthetics of or cause irritation to mammalian skin.

Carriers suitable for use with the present invention include, for example, those used in the formulation of a wide variety of product types, including creams, dispersions, emulsions, gels, lotions, milks, mousses, sprays, and tonics.

The carriers used herein can include a wide range of components conventionally used in cosmetic/dermatological compositions. The carriers can contain a solvent to dissolve or disperse the polymer with C₁-C₆ alcohols or polyhydroxyalcohols, such as ethanol, glycerol, isopropanol, methanol, and mixtures thereof. The carriers can also contain a wide variety of additional materials including, but not limited to, acetone, esters (such as ethyl acetate, dibutyl phthalate), halogenated hydrocarbons (such as freons), hydrocarbons (such as decene, hexane, and isobutane), linalool, and volatile silicon derivatives (especially siloxanes such as phenyl pentamethyl disiloxane, methoxypropyl heptamethyl cyclotetrasiloxane, chloropropyl pentamethyl disiloxane, hydroxypropyl pentamethyl disiloxane, octamethyl cyclotetrasiloxane, decamethyl cyclopentasiloxane, cyclomethicone, dimethicone), and mixtures thereof.

When the composition is a gel, mousse, or tonic the preferred solvents include ethanol, volatile silicone derivatives, and mixtures thereof. The solvents used is such admixtures may be miscible or immiscible with each other. Mousses and aerosol sprays can also utilize any of the conventional propellants to deliver the material as a foam, in the case of a mousse, or as a fine, uniform spray, in the case of an aerosol spray. Examples of suitable propellants include materials such as dichlorodifluoromethane, difluoroethane, dimethylether, isobutane, n-butane, propane, or trichlorofluromethane. A tonic or spray product having a low viscosity may also utilize an emulsifying agent. Examples of suitable emulsifying agents include anionic surfactants, cationic surfactants, nonionic surfactants, and mixtures thereof. Fluorosurfactants are especially preferred, particularly if the product is a spray composition and most especially if it is a spray composition having a relatively low level of volatile organic solvents, such as alcohols, and relatively high levels of water (i.e., in excess of about 10%, by weight, water). If such an emulsifying agent is used, it is preferably present at a level of from about 0.01% to about 7.5% by weight of the composition. The level of propellant can be adjusted as desired, but is generally from about 3% to about 30% by weight of mousse compositions and from about 15% to about 50% by weight of the aerosol spray compositions.

Suitable spray compositions are well known in the art and include conventional, non-aerosol pump sprays, i.e., "atomizers," aerosol containers or cans having propellant, as described above, and also pump aerosol containers utilizing compressed air as the propellant. Pump aerosol containers are disclosed, for example, in U.S. Patent No. 4,077,441, issued to Olofsson on March 7, 1978, and U.S. Patent No. 4,850,577, issued to TerStege on July 25, 1989.

A wide variety of additional components can be employed in the topical cosmetic/dermatological compositions herein. The compositions of the present invention can comprise a safe and effective amount of a pharmaceutical additive or adjuvant. The phrase "safe and effective" means an amount of an active agent high enough to significantly or positively modify the condition to be treated, but low enough to avoid serious side effects (at a reasonable benefit/risk ratio) within the scope of sound medical judgment. A safe and effective amount of the pharmaceutical active agent will vary with the specific active species, the ability of the composition to penetrate the active species through the skin, the amount of composition to be applied, the particular condition being treated, the age and physical condition of the patient being treated, the severity of the condition, the duration of the treatment, the nature of concurrent therapy, and like factors.

Useful pharmaceutical active agents in the present invention include antimicrobial drugs: antibacterials, antifungals, antiprotozoans, and antivirals. Antimicrobial drugs preferred for inclusion in compositions of the present invention include pharmaceutically acceptable salts of β-lactam drugs, amanfadine, amikacin, capreomycin, chlorhexidine, chlortetracycline, ciprofloxacin, clindamycin, doxycycline, erythromycin, ethambutol, gentamicin, kanamycin, lineomycin, methacycline, methenamine, metronidazole, miconazole, minocycline, neomycin, netilmicin, norfloxacin, oxytetracycline, paramomycin, pentamidine, quinolone drugs, streptomycin, tetracycline, tobramycin, and triclosan.

The subject cosmetic/dermatological compositions can contain various emulsifiers when formulated as emulsions. These emulsifiers are useful for emulsifying the various carrier components of the compositions herein. Suitable emulsifiers can include any of a wide variety of nonionic, cationic, anionic, and zwitterionic emulsifiers disclosed in the prior patents and other references. See McCutcheon's, Detergents and Emulsifiers, North American Edition (1986), published by Allured Publishing Corporation; U.S. Patent No. 5,011,681, issued to Ciotti et al. on April 30, 1991; U.S. Patent No. 4,421,769, issued to Dixon et al. on December 20, 1983; and U.S. Patent No. 3,755,560, issued to Dickert et al. on August 28, 1973.

Suitable emulsifier types include acyl lactylates, alkyl phosphates, carboxylic acid copolymers, esters and ethers of glucose, esters of glycerin, esters of propylene glycol, esters of sorbitan anhydrides, esters of sorbitol, ethoxylated ethers, ethoxylated alcohols, fatty acid amides, fatty acid esters of polyethylene glycol, fatty esters of polypropylene glycol, polyoxyethylene fatty ether phosphates, soaps and mixtures thereof.

Preferred emulsifiers can include, but are not limited to, ceteareth-20, ceteth-10, cetyl phosphate, diethanolamine cetyl phosphate, glyceryl stearate, PEG-100 stearate, polyethylene glycol 20 sorbitan monolaurate, polyethylene glycol 5 soya sterol, polysorbate 60, polysorbate 80, potassium cetyl phosphate, PPG-2 methyl glucose ether distrearate, steareth-20, and mixtures thereof.

Typically preferred among these emulsifiers which are useful in the compositions of the present inventions are one or more of the following: oxyethylenated (10 OE) behenyl alcohol and oxyethylenated (4 OE) lauryl alcohol. Combinations of these emulsifiers is similarly preferred.

The subject cosmetic/dermatological compositions can also contain various emollients. Examples of suitable emollients include, but are not limited to, highly branched hydrocarbons, non-polar carboxylic acid and alcohol esters, volatile and non-volatile silicone oils, and mixtures thereof. See, U.S. Patent No. 4,919,934, issued to Deckner et al. on April 24, 1990.

Typically preferred among these emollients which are useful in the compositions of the present inventions are one or more of the following: dicaprylyl ether, dioctyl cyclohexane.

A variety of additional components can be incorporated into the cosmetic/dermatological compositions contained herein. Non-limiting examples of these additional components include cationic polymers and thickeners, chelators, gums and thickeners, low pH thickening agents, polymers for enhancing the film-forming properties and substantivity of the composition, sequestrants, skin penetrating aids, suspending agents, vitamins and derivatives thereof, preservatives and aesthetic components.

Exemplary preservatives, which are conventional in this art and which prevent or retard microbial growth and thus protect cosmetic products from spoilage, are set forth at CFTA International Cosmetic Ingredient Dictionary and Handbook, seventh edition, 2, 1654 (1997).

The cosmetic/dermatological compositions of the present invention are administered in conventional fashion to provide the desired benefit. Such methods of use generally involve application of an effective amount of the composition onto the skin, which then is allowed to remain until absorbed into or removed from the skin. Preferably, creams, dispersions, emulsions, gels, lotions, milks, mousses, sprays, tonics and like are applied to dry mammalian skin and/or scalp.

In order to further illustrate the present invention and the advantages thereof, the following specific examples are given, it being understood that same are intended only as illustrative and in nowise limitative.

### EXAMPLES 1-5

**General preparation of sunscreen/self-tanning compositions:**

| | w/w % |
|---|---|
| **Phase A:** | |
| Oxyethylenated (10 OE) behenyl alcohol | 2.50 |
| Oxyethylenated (4 OE) lauryl alcohol | 2.50 |
| Dicaprylyl ether | 2.75 |
| Dioctyl cyclohexane | 2.50 |
| Parsol 1789 (Avobenzone) | 3.00 |
| Octyl salicylate | 5.00 |
| Octocrylene | 10.00 |
| Oxybenzone | 6.00 |
| Preservative | 1.00 |
| Polymer | 1.00 |

| **Phase B:** | |
|---|---|
| Glycerol | 2.50 |
| Water | 32.00 |
| Hexadecylic phosphate alcohol, K salt | 0.30 |

| **Phase C:** | |
|---|---|
| Dihydroxyacetone | 5.00 |
| Water | qs 100 |

The ingredients of Phase B were introduced into a main tank and heated with homogenization to 80-85° C. The ingredients of Phase A were combined in a separate tank ("side tank")and heated with moderate propeller mixing to 80°-85° C. Once the temperature of the contents of both tanks was 80°-85° C and homogeneous, the contents of the side tank were added to the main tank. The entire contents of the main tank were mixed with increased homogenization for 15 to 20 minutes. Cooling was begun and homogenization was decreased. Once the contents of the main tank reached 35° C, the contents of Phase C were added to the main tank. Once the contents of the main tank reached 25° C, homogenization and cooling were ceased. This bulk was then passed through a High Pressure Homogenizer three times at 500 bar pressure.

The following polymers were incorporated into the cosmetic/dermatological compositions as indicated above: Polymer 1 was Performa V1608 (C30-38 olefin/isopropyl maleate/MA copolymer) of New Phase Technologies, 377 Hoes Lane, Suite 120, Piscataway, New Jersey, 08854, U.S.A.; Polymer 2 was Ganex V220 Antaron V220 (vinylpyrrolidone/eicosene copolymer) of International Specialty Products, 1361 Alps Road, Wayne, New Jersey, 07470, U.S.A.; Polymer 3 was Cosmacol ETLP (dimyristyl tartrate) of Condea Augusta S.P.A., Via Medici del Vascello, 26-20138, Milan, Italy; Polymer 4 was SA 70-5 in solution in decamethylcyclopentasiloxane (silicone/acrylic) of Minnesota Mining and Manufacturing, 3M Center, Building 275-5E-08, St. Paul, Minnesota, 55144-1000, U.S.A.

### Test of Sun Protection Factor:

The composition containing Polymer 2 was not evaluated because an unstable emulsion was obtained twenty-four hours after batching. It appeared as though Polymer 2 destroyed the dispersion of the internal (oil) phase in the external (water) phase.

The protection factor on the skin against UV radiation was determined relative to each of the remaining compositions 1 to 4 containing polymers 1, 3, and 4, as described above, and composition 5, which contained no polymer.

| | |
|---|---|
| **Composition Number -Polymer Used** | **Sun Protection Factor** |
| 1 - Polymer 1 | 19.60 |
| 2 - Polymer 2 | - |
| 3 - Polymer 3 | 12.20 |
| 4 - Polymer 4 | 25.35 |
| 5 - No Polymer | 9.35 |

These results plainly evidence that the sunscreen/self-tanning composition according to the invention, containing Polymer 4, had an average sun protection factor which was considerably higher than that of the sunscreen/self-tanning compositions containing different polymers or no polymer.

Polymer 1 provided the next highest sun protection factor to Polymer 4. However, after only two months of storage, the compositions containing Polymer 1 were unstable. The compositions containing Polymer 4, after two months of storage, remained stable. Therefore, the sunscreen-self-tanning compositions according to the invention containing Polymer 4 provide superior advantages.

## Claims

1. A topically applicable cosmetic/dermatological composition suited for both the artificial tanning as well as the UV-photoprotection of the skin and/or scalp of a mammalian organism, comprising: (i) an effective UV-photoprotecting amount of at least one UV-photoprotecting agent, (ii) an effective artificial tanning amount of at least one artificial tanning agent, and (iii) a compatibilizing amount of poly(isobutyl methacrylate-co-methyl FOSEA)-g-poly(dimethylsiloxane) or of a terpolymer of isobutyl methacrylate, acrylic acid and a methacryloxypropyl/butyldimethyl silyl macromer and wherein the said composition is formulated as emulsion and contains at least one emulsifier.

2. The cosmetic/dermatological composition as defined by Claim 1, wherein the artificial tanning agent comprises dihydroxyacetone.

3. The cosmetic/dermatological composition as defined by Claim 2, said UV-photoprotecting agent comprising at least one cerium oxide, chromium oxide, cobalt oxide, iron oxide, red petrolatum, silicone-treated titanium dioxide, titanium dioxide, zinc oxide, or zirconium oxide.

4. The cosmetic/dermatological composition as defined by Claim 3, wherein the UV-photoprotecting agent comprises titanium dioxide.

5. The cosmetic/dermatological composition as defined by Claim 1, said UV-photoprotecting agent comprising at least one p-aminobenzoic acid derivative, anthranilate, benzophenone, camphor derivative, cinnamic derivative, dibenzoyl methane compound, β,β-diphenylacrylate derivative, salicylic derivative, triazine derivative, benzimidazole compound, bis-benzoazolyl derivative, methylene bis-(hydroxyphenylbenzotriazole) compound, sunscreen polymer, sunscreen silicone, or mixture thereof.

6. The cosmetic/dermatological composition as defined by Claim 5, wherein the UV-photoprotecting agent comprises a camphor derivative.

7. The cosmetic/dermatological composition as defined by Claim 5, wherein the UV-photoprotecting agent comprises at least one of avobenzone, octyl salicylate, octocrylene, and oxybenzone.

8. The cosmetic/dermatological composition as defined in any of Claims 1 to 7, wherein the emulsion is an oil-in-water emulsion, a water-in-oil emulsion, a water-in-oil-in-water emulsion or an oil-in-water-in-silicone emulsion.

9. The cosmetic/dermatological composition as defined in any of Claims 1 to 8, wherein the emulsifier is selected from the group consisting of acyl lactylates, alkyl phosphates, carboxylic acid copolymers, esters and ethers of glucose, esters of glycerin, esters of propylene glycol, esters of sorbitan anhydrides, esters of sorbitol, ethoxylated ethers, ethoxylated alcohols, fatty acid amides, fatty acid esters of polyethylene glycol, fatty esters of polypropylene glycol, polyoxyethylene fatty ether phosphates, soaps and mixtures thereof.

10. The cosmetic/dennatological composition as defined by Claim 9, wherein the emulsifiers is selected from the group consisting of ceteareth-20, ceteth-10, cetyl phosphate, diethanolamine cetyl phosphate, glyceryl stearate, PEG-100 stearate, polyethylene glycol 20 sorbitan monolaurate, polyethylene glycol 5 soya sterol, polysorbate 60, polysorbate 80, potassium cetyl phosphate, PPG-2 methyl glucose ether distearate, steareth-20, and mixtures thereof.

11. The cosmetic/dermatological composition as defined by any Claims 8 to 10 , wherein the emulsifiers is one or more of the following: oxyethylenated (10 OE) behenyl alcohol and oxyethylenated (4 OE) lauryl alcohol and preferably their mixture.

12. A method for the preparation of a dermatological composition as defined in any of Claim 1 to 11 for artificially tanning and photoprotecting mammalian skin and/or scalp against the deleterious effects of ultraviolet irradiation, comprising topically applying onto the skin and/or scalp of a mammalian subject.

13. A method for compatibilizing and/or stabilizing a cosmetic/dermatological composition comprising at least one UV-photoprotecting agent and at least one artificial tanning agent and which is formulated as emulsion and containing at least one emulsifier, the said method comprising admixing and formulating into such composition a thus-effective amount of poly(isobutyl methacrylate-co-methyl FOSEA)-g-poly(dimethylsiloxane) or of a terpolymer of isobutyl methacrylate, acrylic acid and a methacryloxypropyl/butyldimethyl silyl macromer.

14. A method according to Claim 13, wherein the artificial tanning agent comprises dihydroxyacetone.

15. A method according to Claim 13, wherein the UV-photoprotecting agent comprises at least one cerium oxide, chromium oxide, cobalt oxide, iron oxide, red petrolatum, silicone-treated titanium dioxide, titanium dioxide, zinc oxide, or zirconium oxide.

16. A method according to Claim 15, wherein the UV-photoprotecting agent comprises titanium dioxide.

17. A method according to Claim 13, wherein said UV-photoprotecting agent comprising at least one p-aminobenzoic acid derivative, anthranilate, benzophenone, camphor derivative, cinnamic derivative, dibenzoyl methane compound, β,β-diphenylacrylate derivative, salicylic derivative, triazine derivative, benzimidazole compound, bis-benzoazolyl derivative, methylene bis-(hydroxyphenylbenzotriazole) compound, sunscreen polymer, sunscreen silicone, or mixture thereof.

18. A method according to Claim 17, wherein the UV-photoprotecting agent comprises a camphor derivative.

19. A method according to Claim 17, wherein the UV-photoprotecting agent comprises at least one of avobenzone, octyl salicylate, octocrylene, and oxybenzone.

20. A method as defined in any of Claims 13 to 19, wherein the emulsion is an oil-in-water emulsion, a water-in-oil emulsion, a water-in-oil-in-water emulsion or an oil-in-water-in-silicone emulsion.

21. A method as defined in any of Claims 13 to 20, wherein the emulsifier is selected from the group consisting of acyl lactylates, alkyl phosphates, carboxylic acid copolymers, esters and ethers of glucose, esters of glycerin, esters of propylene glycol, esters of sorbitan anhydrides, esters of sorbitol, ethoxylated ethers, ethoxylated alcohols, fatty acid amides, fatty acid esters of polyethylene glycol, fatty esters of polypropylene glycol, polyoxyethylene fatty ether phosphates, soaps and mixtures thereof.

22. A method as defined by Claim 21, wherein the emulsifiers is selected from the group consisting of ceteareth-20, ceteth-10, cetyl phosphate, diethanolamine cetyl phosphate, glyceryl stearate, PEG-100 stearate, polyethylene glycol 20 sorbitan monolaurate, polyethylene glycol 5 soya sterol, polysorbate 60, polysorbate 80, potassium cetyl phosphate, PPG-2 methyl glucose ether distearate, steareth-20, and mixtures thereof.

23. A method as defined by any Claims 13 to 22, wherein the emulsifiers is one or more of the following: oxyethylenated (10 OE) behenyl alcohol and oxyethylenated (4 OE) lauryl alcohol and preferably their mixture.

## Patentansprüche

1. Topisch anwendbare kosmetische/dermatologische Zusammensetzung, die zur künstlichen Bräunung sowie zum UV-Lichtschutz der Haut und/oder des Kopfhaars eines Säugeorganismus geeignet ist und enthält:
(i) eine zum UV-Lichtschutz wirksame Menge mindestens eines UV-Lichtschutzmittels,
(ii) eine zur künstlichen Bräunung wirksame Menge mindestens eines Mittels zur künstlichen Bräunung
und
(iii) eine kompatibilisierende Menge Poly(isobutylmethacrylat-co-methyl FOSEA)-g-poly(dimethylsiloxan) oder eines Terpolymers von Isobutylmethacrylat, Acrylsäure und einem Methacryloxypropyl/butyldimethylsilyl-Makromer,
wobei die Zusammensetzung als Emulsion formuliert ist und mindestens einen Emulgator enthält.

2. Kösmetische/dermatologische Zusammensetzung nach Anspruch 1, bei der das Mittel zur künstlichen Bräunung Dihydroxyaceton ist.

3. Kosmetische/dermatologische Zusammensetzung nach Anspruch 2, bei der das UV-Lichtschutzmittel mindestens ein Stoff ist, der ausgewählt ist unter Ceroxiden, Chromoxiden, Cobaltoxiden, Eisenoxiden, rotem Petrolatum, Silicon-behandeltem Titandioxid, Titandioxid, Zinkoxid und Zirconiumoxid.

4. Kosmetische/dermatologische Zusammensetzung nach Anspruch 3, bei der das UV-Lichtschutzmittel Titandioxid ist.

5. Kosmetische/dermatologische Zusammensetzung nach Anspruch 1, bei der das UV-Lichtschutzmittel mindestens ein Stoff ist, der ausgewählt ist unter p-Aminobenzoesäure-Derivaten, Anthranilaten, Benzophenon, Campherderivaten, Zimtsäurederivaten, Dibenzoylmethan-Verbindungen, β,β-Diphenylacrylat-Derivaten, Salicylsäure-Derivaten, Triazin-Derivaten, Benzimidazol-Verbindungen, Bis-benzoazolyl-Derivaten, Methylen-bis(hydroxyphenylbenzotriazol)-Verbindungen, Lichtschutzpolymeren und Lichtschutzsiliconen sowie Gemischen dieser Stoffe.

6. Kosmetische/dermatologische Zusammensetzung nach Anspruch 5, bei der das UV-Lichtschutzmittel ein Campherderivat ist.

7. Kosmetische/dermatologische Zusammensetzung nach Anspruch 5, bei der das UV-Lichtschutzmittel mindestens ein Stoff ist, der unter Avobenzone, Octylsalicylat, Octocrylene und Oxybenzone ausgewählt ist.

8. Kosmetische / dermatologische Zusammensetzung nach einem oder mehreren der Ansprüche 1 bis 7, bei der die Emulsion eine Öl-in-Wasser-Emulsion, eine Wasser-in-Öl-Emulsion, eine Wasser-in-Öl-in-Wasser-Emulsion oder eine Öl-in-Wasser-in-Silicon-Emulsion ist.

9. Kosmetische/dermatologische Zusammensetzung nach einem oder mehreren der Anspruche 1 bis 8, bei der der Emulgator ausgewählt ist aus der Gruppe, die besteht aus Acyllactylaten, Alkylphosphaten, Carboxylgruppen-enthaltenden Copolymeren, Estern und Ethern von Glucose, Glycerinestern, Estern von Propylenglykol, Estern von Sorbitananhydriden, Sorbitestern, ethoxylierten Ethern, ethoxylierten Alkoholen, Fettsäureamiden, Fettsäureestern von Polyethylenglykol, Fettsäureestern von Polypropylenglykol, Polyoxyethylen-Fettsäureetherphosphaten und Seifen sowie Gemischen dieser Stoffe.

10. Kosmetische/dermatologische Zusammensetzung nach Anspruch 9, bei welcher der Emulgator aus der Gruppe ausgewählt ist, die besteht aus Ceteareth-20, Ceteth-10, Cetylphosphat, Diethanolamin-cetylphosphat, Glycerylstearat, PEG-100 Stearate, Polyethylenglycol 20 Sorbitan Monolaurate, Polyethylenglycol 5 Soya Sterol, Polysorbat 60, Polysorbat 80, Kaliumcetylphosphat, PPG-2 Methyl Glucose Ether Distearate und Steareth-20 sowie Gemischen dieser Stoffe.

11. Kosmetische/dermatologische Zusammensetzung nach einem oder mehreren der Anspruche 8 bis 10, bei welcher der Emulgator einer oder mehrere der folgenden Stoffe ist: ethoxylierter (10 EO) Behenylalkohol und ethoxylierter (4 EO) Laurylalkohol und bevorzugt ein Gemisch davon.

12. Verfahren zur Herstellung einer dermatologischen Zusammensetzung nach einem oder mehreren der Ansprüche 1 bis 11 zur künstlichen Bräunung sowie zum Lichtschutz der Haut und/oder des Kopfhaars von Säugern gegen schädliche Wirkungen ultravioletter Strahlung, das die topische Anwendung auf der Haut und/ oder dem Kopfhaar eines Säugerorganismus umfasst.

13. Verfahren zur Kompatibilisierung und/oder Stabilisierung einer kosmetischen/dermatologischen Zusammensetzung, die mindestens ein UV-Lichtschutzmittel und mindestens ein Mittel zur künstlichen Bräunung enthält, als Emulsion formuliert ist und mindestens einen Emulgator enthält, wobei das Verfahren den Zusatz einer dementsprechend wirksamen Menge Poly(isobutylmethacrylat-co-methyl FOSEA)-g-poly(dimethylsiloxan) oder eines Terpolymers von Isobutylmethacrylat, Acrylsäure und einem Methacryloxypropyl/butyldimethylsilyl-Makromer und die Formulierung zu einer solchen Zusammensetzung umfasst.

14. Verfahren nach Anspruch 13, bei dem das Mittel zur künstlichen Bräunung Dihydroxyaceton ist.

15. Verfahren nach Anspruch 13, bei dem das UV-Lichtschutzmittel mindestens ein Stoff ist, der ausgewählt ist unter Ceroxiden, Chromoxiden, Cobaltoxiden, Eisenoxiden, rotem Petrolatum, Silicon-behandeltem Titandioxid, Titandioxid, Zinkoxid und Zirconiumoxid.

16. Verfahren nach Anspruch 15, bei dem das UV-Lichtschutzmittel Titandioxid ist.

17. Verfahren nach Anspruch 13, bei dem das UV-Lichtschutzmittel mindestens ein Stoff ist, der ausgewählt ist unter p-Aminobenzoesäure-Derivaten, Anthranilaten, Benzophenon, Campherderivaten, Zimtsäurederivaten, Dibenzoylmethan-Verbindungen, β,β-Diphenylacrylat-Derivaten, Salicylsäure-Derivaten, Triazin-Derivaten, Benzimidazol-Verbindungen, Bis-benzoazolyl-Derivaten, Methylen-bis(hydroxyphenylbenzotriazol)-Verbindungen, Lichtschutzpolymeren und Lichtschutzsiliconen sowie Gemischen dieser Stoffe.

18. Verfahren nach Anspruch 17, bei dem das UV-Lichtschutzmittel ein Campherderivat ist.

19. Verfahren nach Anspruch 17, bei dem das UV-Lichtschutzmittel mindestens ein unter Avobenzone, Octylsalicylat, Octocrylene und Oxybenzone ausgewählter Stoff ist.

20. Verfahren nach einem oder mehreren der Ansprüche 13 bis 19, bei dem die Emulsion eine Öl-in-Wasser-Emulsion, eine Wasser-in-Öl-Emulsion, eine Wasser-in-Öl-in-Wasser-Emulsion oder eine Öl-in-Wasser-in-Silicon-Emulsion ist.

21. Verfahren nach einem oder mehreren der Ansprüche 13 bis 20, bei dem der Emulgator aus der Gruppe ausgewählt ist, die besteht aus Acyllactylaten, Alkylphosphaten, Carboxylgruppen-enthaltenden Copolymeren, Estern und Ethern von Glucose, Glycerinestern, Estern von Propylenglykol, Estern von Sorbitananhydriden, Sorbitestern, ethoxylierten Ethern, ethoxylierten Alkoholen, Fettsäureamiden, Fettsäureestern von Polyethylenglykol, Fettsäureestern von Polypropylenglykol, Polyoxyethylen-Fettsäureetherphosphaten und Seifen sowie Gemischen dieser Stoffe.

22. Verfahren nach Anspruch 21, bei dem der Emulgator aus der Gruppe ausgewählt ist, die besteht aus Ceteareth-20, Ceteth-10, Cetylphosphat, Diethanolamin-cetylphosphat, Glycerylstearat, PEG-100 Stearate, Polyethylenglycol 20 Sorbitan Monolaurate, Polyethylenglycol 5 Soya Sterol, Polysorbat 60, Polysorbat 80, Kaliumcetylphosphat, PPG-2 Methyl Glucose Ether Distearate und Steareth-20 sowie Gemischen dieser Stoffe.

23. Verfahren nach einem oder mehreren der Ansprüche 13 bis 22, bei dem der Emulgator eine oder mehrere der folgenden Verbindungen ist: ethoxylierter (10 EO) Behenylalkohol und ethoxylierter (4 EO) Laurylalkohol und bevorzugt ein Gemisch davon.

## Revendications

1. Composition cosmétique/dermatologique applicable par voie topique adaptée pour le bronzage artificiel ainsi que pour la photoprotection anti-UV de la peau et/ou du cuir chevelu d'un organisme mammalien, comprenant : (i) une quantité efficace photoprotectrice anti-UV d'au moins un agent photoprotecteur anti-UV, (ii) une quantité efficace pour bronzage artificiel d'au moins un agent de bronzage artificiel, et (iii) une quantité compatibilisante de poly(méthacrylate d'isobutyle-co-FOSEA de méthyle)-g-poly(diméthylsiloxane) ou d'un terpolymère de méthacrylate d'isobutyle, d'acide acrylique et d'un macromère de méthacryloxypropyle/ butyldiméthylsilyle, et dans laquelle ladite composition est formulée sous forme d'une émulsion et contient au moins un émulsifiant.

2. Composition cosmétique/dermatologique telle que définie selon la revendication 1, dans laquelle l'agent de bronzage artificiel comprend de la dihydroxyacétone.

3. Composition cosmétique/dermatologique telle que définie selon la revendication 2, ledit agent photoprotecteur anti-UV comprenant au moins un parmi l'oxyde de cérium, l'oxyde de chrome, l'oxyde de cobalt, l'oxyde de fer, le pétrolatum rouge, le dioxyde de titane siliconé, le dioxyde de titane, l'oxyde de zinc ou l'oxyde de zirconium.

4. Composition cosmétique/dermatologique telle que définie selon la revendication 3, dans laquelle l'agent photoprotecteur anti-UV comprend du dioxyde de titane.

5. Composition cosmétique/dermatologique telle que définie selon la revendication 1, ledit agent photoprotecteur anti-UV comprenant au moins parmi un dérivé de l'acide p-aminobenzoïque, un anthranilate, une benzophénone, un dérivé de camphre, un dérivé cinnamique, un composé de dibenzoylméthane, un dérivé de β,β-diphénylacrylate, un dérivé salicylique, un dérivé de triazine, un composé de benzimidazole, un dérivé de bis-benzoazolyle, un composé de méthylène bis-(hydroxyphénylbenzotriazole), un polymère de filtre solaire, un silicone de filtre solaire, ou un mélange de ceux-ci.

6. Composition cosmétique/dermatologique telle que définie selon la revendication 5, dans laquelle l'agent photoprotecteur anti-UV comprend un dérivé de camphre.

7. Composition cosmétique/dermatologique telle que définie selon la revendication 5, dans laquelle l'agent photoprotecteur anti-UV comprend au moins un parmi l'avobenzone, le salicylate d'octyle, l'octocrylène et l'oxybenzone.

8. Composition cosmétique/dermatologique telle que définie selon l'une quelconque des revendications 1 à 7, dans laquelle l'émulsion est une émulsion huile-dans-eau, une émulsion eau-dans-huile, une émulsion eau-dans-huile-dans-eau ou une émulsion huile-dans-eau-dans-silicone.

9. Composition cosmétique/dermatologique telle que définie selon l'une quelconque des revendications 1 à 8, dans laquelle l'émulsifiant est choisi parmi le groupe constitué par les lactylates d'acyle, les phosphates d'alkyle, les copolymères d'acides carboxyliques, les esters et les éthers de glucose, les esters de glycérine, les esters de propylène glycol, les esters d'anhydrides de sorbitan, les esters de sorbitol, les éthers éthoxylés, les alcools éthoxylés, les amides d'acides gras, les esters d'acide gras et de polyéthylène glycol, les esters gras de polypropylène glycol, les phosphates d'éther gras et de polyoxyéthylène, les savons et des mélanges de ceux-ci.

10. Composition cosmétique/dermatologique telle que définie selon la revendication 9, dans laquelle l'émulsifiant est choisi parmi le groupe constitué par le cétéareth-20, le céteth-10, le phosphate de cétyle, le cétylphosphate de diéthanolamine, le stéarate de glycéryle, le stéarate de PEG-100, le monolaurate de polyéthylène glycol 20 et de sorbitan, le stérol de soja et de polyéthylène glycol 5, le polysorbate 60, le polysorbate 80, le phosphate de cétyle potassique, le distéarate d'éther de méthylglucose et de PPG-2, le stéareth-20, et des mélanges de ceux-ci.

11. Composition cosmétique/dermatologique telle que définie selon l'une quelconque des revendications 8 à 10, dans laquelle l'émulsifiant est l'un ou plusieurs parmi ce qui suit : l'alcool béhénylique oxyéthyléné (10 OE) et l'alcool laurylique oxyéthyléné (4 OE) et préférablement leur mélange.

12. Méthode de préparation d'une composition dermatologique telle que définie selon l'une quelconque des revendications 1 à 11, destinée au bronzage artificiel et à la photoprotection de la peau et/ou du cuir chevelu mammalien contre les effets néfastes d'un rayonnement ultraviolet, comprenant l'application par voie topique sur la peau et/ou le cuir chevelu d'un sujet mammalien.

13. Méthode de compatibilisation et/ou de stabilisation d'une composition cosmétique/dermatologique comprenant au moins un agent photoprotecteur anti-UV et au moins un agent de bronzage artificiel et qui est formulée sous forme d'une émulsion et contenant au moins un émulsifiant, ladite méthode comprenant le co-mélange et la formulation en une telle composition d'une quantité ainsi efficace de poly(méthacrylate d'isobutyle-co-FOSEA de méthyle)-g-poly(diméthyl-siloxane) ou d'un terpolymère de méthacrylate d'isobutyle, d'acide acrylique et d'un macromère de méthacryloxypropyle/butyldiméthylsilyle.

14. Méthode selon la revendication 13, dans laquelle l'agent de bronzage artificiel comprend de la dihydroxyacétone.

15. Méthode selon la revendication 13, dans laquelle l'agent photoprotecteur anti-UV comprend au moins un parmi l'oxyde de cérium, l'oxyde de chrome, l'oxyde de cobalt, l'oxyde de fer, le pétrolatum rouge, le dioxyde de titane siliconé, le dioxyde de titane, l'oxyde de zinc ou l'oxyde de zirconium.

16. Méthode selon la revendication 15, dans laquelle l'agent photoprotecteur anti-UV comprend du dioxyde de titane.

17. Méthode selon la revendication 13, dans laquelle ledit agent photoprotecteur anti-UV comprend au moins un parmi un dérivé de l'acide p-aminobenzoïque, un anthranilate, une benzophénone, un dérivé de camphre, un dérivé cinnamique, un composé de dibenzoylméthane, un dérivé de β,β-diphénylacrylate, un dérivé salicylique, un dérivé de triazine, un composé de benzimidazole, un dérivé de bis-benzoazolyle, un composé de méthylène bis-(hydroxyphénylbenzotriazole), un polymère de filtre solaire, un silicone de filtre solaire, ou un mélange de ceux-ci.

18. Méthode selon la revendication 17, dans laquelle l'agent photoprotecteur anti-UV comprend un dérivé de camphre.

19. Méthode selon la revendication 17, dans laquelle l'agent photoprotecteur anti-UV comprend au moins un parmi l'avobenzone, le salicylate d'octyle, l'octocrylène et l'oxybenzone.

20. Méthode telle que définie selon l'une quelconque des revendications 13 à 19, dans laquelle l'émulsion est une émulsion huile-dans-eau, une émulsion eau-dans-huile, une émulsion eau-dans-huile-dans-eau ou une émulsion huile-dans-eau-dans-silicone.

21. Méthode telle que définie selon l'une quelconque des revendications 13 à 20, dans laquelle l'émulsifiant est choisi parmi le groupe constitué par les lactylates d'acyle, les phosphates d'alkyle, les copolymères d'acides carboxyliques, les esters et les éthers de glucose, les esters de glycérine, les esters de propylène glycol, les esters d'anhydrides de sorbitan, les esters de sorbitol, les éthers éthoxylés, les alcools éthoxylés, les amides d'acides gras, les esters d'acide gras et de polyéthylène glycol, les esters gras de polypropylène glycol, les phosphates d'éther gras et de polyoxyéthylène, les savons et des mélanges de ceux-ci.

22. Méthode telle que définie selon la revendication 21, dans laquelle l'émulsifiant est choisi parmi le groupe constitué par le cétéareth-20, le céteth-10, le phosphate de cétyle, le cétylphosphate de diéthanolamine, le stéarate de glycéryle, le stéarate de PEG-100, le monolaurate de polyéthylène glycol 20 et de sorbitan, le stérol de soja et de polyéthylène glycol 5, le polysorbate 60, le polysorbate 80, le phosphate de cétyle potassique, le distéarate d'éther de méthylglucose et de PPG-2, le stéareth-20, et des mélanges de ceux-ci.

23. Méthode telle que définie selon l'une quelconque des revendications 13 à 22, dans laquelle l'émulsifiant est l'un ou plusieurs parmi ce qui suit : l'alcool béhénylique oxyéthyléné (10 OE) et l'alcool laurylique oxyéthyléné (4 OE) et préférablement leur mélange.
